Europäisches Patentamt

European Patent Office

Office européen des brevets

(11) Publication number: **0 467 432 A1**

# EUROPEAN PATENT APPLICATION

(21) Application number: **91201551.8**

(22) Date of filing: **19.06.91**

(51) Int. Cl.5: **A61B 5/00**

(30) Priority: **20.07.90 NL 9001660**

(43) Date of publication of application:
**22.01.92 Bulletin 92/04**

(84) Designated Contracting States:
**AT BE CH DE DK ES FR GB GR IT LI NL SE**

(71) Applicant: **LAICA LASER TECHNOLOGY VENTURES B.V.**
**Nieuwe Achtergracht 127**
**NL-1018 WS Amsterdam(NL)**

(72) Inventor: **van Gemert, Martinus Johannes Coenraad**
**Galgenwaard 19**
**NL-3261 LW Breukelen(NL)**
Inventor: **Sterenborg, Henrikus Josephus Cornelis Maria**
**Vredelust 28**
**NL-1111 VC Diemen(NL)**
Inventor: **Beek, Johan Freerk**
**Jacob van Lennepkade 59**
**NL-1054 ZH Amsterdam(NL)**

(74) Representative: **Flamman, Han et al**
**LIOC Patents and Licensing P.O. Box 85096**
**NL-3508 AB Utrecht(NL)**

(54) **Device for detecting air in the pleural cavity.**

(57) A device for detecting air in the pleural cavity of a human being or an animal contains at least one lightsource (1), which emits light via a photo-conductive fibre (2) onto one or more places on the outside of the chest wall (4), situated in front of the pleural cavity (6), The device further contains one or more photo sensors (8) to detect the light scattered from the edge surfaces of the pleural cavity (6) at one or more places on the outside of the chest-wall (4), and an electronic device (10) for processing the signals (9) emanating from the sensors (8).

FIGURE 1

The invention relates to a device for detecting air in the pleural cavity of a human being or an animal. Under normal physiological circumstances there is no air situated in the pleural cavity outside of the airways (tractus respiratorus). A device to detect the introduction of air into the pleural cavity is in itself unknown. It is known, that pressure changes in the pleural cavity which accompany the introduction of air in the pleural cavity can be deduced in a known manner from changes in the intraesophageal pressure, as is described by H. Korvenranta and P. Kero in "Intraesophageal pressure monitoring in infants with respiratory disorders" in Crit. Care Med. 1983; 11 (4): P276-79.

According to this method a pressure catheter is introduced into the gullet (oesophagus) of the patient who is to be examined. The method described is only carried out for the benefit of scientific purposes; the invasive method is relatively taxing for a patient and is unsuitable for application as a routine surveillance of patients who are eligible to be kept under control.

The importance of a device for detecting air in the pleural cavity is especially situated in the possibility to notice the possible occurrence of a pneumothorax, also known as a "collapsed lung", in good time. A pneumothorax constitutes an important respiratory complication in neonatology. The pathophysiological mechanism which is at the basis of this complication is the fact that several lung blisters (alveoli) burst towards the cavity in which the lungs are situated under the influence of respiratory pressure. Under physiological circumstances there is an under-pressure in this cavity (the pleural cavity). Thus under-pressure ensures that the elastic lung remains in a nice unfolded position. Removing this under-pressure will result in a certain collapse of the elastic tissue of the lung. In the case of a collapsed lung one speaks of a pneumothorax. This phenomenum can occur both on a single and on both sides.

The pneumothorax which arises in newborn children as a respiratory complication is, in general, a valve or tension pneumothorax. This involves air which has entered the pleural cavity not being able to escape. The pressure will rise and the lung can also be compromised on the contra-lateral side, together with the heart. Furthermore, a torsion of the circulatory system can occur and intracranial hemorrhages can arise (as an indirect consequence).

Of the newborn children who have to be respirated, 10 to 35% develop a pneumothorax as a complication of the respiration.

It is, in itself, simple to diagnose a pneumothorax with an x-ray photograph or through trans-illumination. The therapy is also relatively simple. Mortality, however, is high: 10 to 20%. This is caused by, among other things, the fact that it is difficult to recognize the clinical image. Recognition of respiratory pathology is hampered by the continuous respiration. Furthermore, it is very difficult to notice a clinical worsening in a child which is already doing so badly that it has to be respirated. Mostly, a receding peripheral oxygen tension provides the first reason for further diagnostics. This results in the fact that a diagnosis as pneumothorax is made at a late stage, and sometimes too late.

The aims of the invention are to provide a device with which air in the pleural cavity is detected in a non-invasive manner and a manner which is not taxing for the patient.

These aims are achieved in accordance with the invention with a device which contains at least one light source which emits light at one or more places onto the outer side of the chest wall which is situated in front of the pleural cavity, directly or via a photo-conductive fibre or an imaging system, which light is scattered and reflected In and from the edge surfaces of the pleural cavity, together with one or more photo-sensitive sensors to detect the scattered and reflected light at one or more places on the outside of the chest wall, together with an electronic device for processing the signals which emanate from the sensors.

The invention is based on the insight that it is possible to emit a light beam into the chest (thorax) of a human being or an animal, and to deduce a (local) change which takes place in the sizes of the pleural cavity, caused by the introduction of air, from the signal which returns as a result of the scattering and reflection of the successive tissue layers and lung tissue.

In an example of a design of a device according to the invention the wave length of the light emitted by the light source amounts to a minimum of 550 nanometres and a maximum of 1200 nanometres. It has been found that light from this range of wave lengths penetrates the relevant tissues sufficiently deeply, while at the same time a sufficient quantity of light is reflected which can be detected by the sensors.

The device functions in an optimal manner if it is equipped with a stable light source with a sufficiently high capacity. In an example of a design of a device according to the invention the light source is therefore a semi-conductor light source, more especially a semi-conductor laser. This latter type of light source offers considerable advantages as a result of its mono-chromatic light, its small size and low power supply.

It has been found that one can suffice with a power density of the transmitted light on the outside of the wall of the pleural cavity (in this case the chest of the patient) of a maximum of 2000 W/m$^2$.

In a following example of a design a sensor and a light source, or the tips of photo-conductive fibres or imaging systems which are connected with that sensor and that light source, are combined in a container.

A very compact container is achieved if the distance between the sensor and the light source, or, as the case may be, the tips of the fibres or the imaging systems, amounts to a maximum of 2 cm, or if the tips coincide. A device according to this last example, in which use is made of the same photo- conductive fibre for the emission of the light and for detecting the reflected and scattered light, has certain advantages from an ergonomic point of view, and is particularly suited, as a result of the small size of the container, for application with new-born babies.

A deviation from normal physiological circumstances as concerns the presence of air in the pleural cavity can be detected with the aid of a device which is characterized by the fact that the electronic device contains a circuit with which the absolute and/or relative deviation of the average value of the amplitude of the signal from the sensors as time progresses is directly determined.

A device which is characterized by the fact that the electronic device contains a circuit with which the absolute and/or relative deviation of the signal from the sensors as time progresses is directly determined also offers the possibility to directly signal the presence of air in the pleural cavity.

Both latter examples of designs are based in the insight that the signal deduced from the sensors (which, for the benefit of a good understanding, is presumed in the following to be an electric charge, an electric current is, however, just as possible) consists of a direct current component, on which an alternating current with the frequency of the respiration of the test person or patient has been superimposed. Under normal physiological conditions the average values of the amplitude of the alternating current component and the direct current do not change, or change very slowly. The introduction of air into the pleural cavity, on the other hand, will become immediately visible as a change in both of the latter values, so that a change can also be seen directly in the absolute and the relative deviation of the average values thereof.

In a following example of a design the electronic device contains a circuit with which the frequency is determined of the signal from the sensors.

In a preferable example of a design the electronic device contains a circuit with which the phase-difference is determined between the signal from the sensors and a reference signal. It is preferable to use a signal deduced from the respiratory frequency of the examined person concerned as a reference signal. Such a reference signal can be deduced, for example, from a flowmeter which is used to register the respiration.

Under normal physiological conditions the phase difference between the signal from the sensors and the reference signal deduced from the respiratory frequency is constant. The introduction of air into the pleural cavity leads to a phase shift, and can therefore be detected with this preferable design.

The invention relates furthermore to an apparatus for detecting a pneumothorax (a pneumothorax detector), based on detection of air in the pleural cavity. Such an apparatus is not known.

The aims of the invention are to provide an apparatus with which patients, especially new-born babies, who are respirated, are monitored continuously as far as the development of a pneumothorax is concerned. As has been explained above, it is of vital importance under certain circumstances to detect the development of a pneumothorax in good time, in order to be able to take suitable measures In order to avoid further complications.

These aims are achieved in accordance with the invention with an apparatus which contains a device for detecting air in the pleural cavity of a human being or an animal according to the invention described above.

With such an apparatus it is possible to monitor a patient continuously according to a non-invasive and non-taxing method.

In the detection apparatus for a pneumothorax a combination of measured and processed signals from the device for detecting the air, which depend on the patient and the medical situation, which are most sensitive for the development of a pneumothorax, can be offered to an electronic circuit in which the measured values are compared with certain threshold values. In the event of transgression of these threshold values or a combination thereof a warning signal is generated.

The possibility of the device for detecting air to deliver information on the development of a pneumothorax are put to use especially in a preferred design of the pneumothorax detector, if the latter contains a semi-conductor laser with a wave length between 550 and 1200 nanometres, if the light source and a sensor, or the tips of fibres or imaging systems connected therewith, with a minimum distance between them, are combined in a container, and if the electronic device contains circuits with which both the phase difference of the signal from the sensors and a reference signal, and the absolute and/or relative deviation in the amplitude with which the signal from a sensor varies and in the average value of the signal, are directly determined.

An example of a design of a pneumothorax detector is characterized by the fact that the value of the phase difference of the signal from the sensors and a reference signal, the value of the absolute and/or relative deviation in the amplitude with which the signal from the sensor varies, and the value of the absolute and/or relative deviation in the average signal from the sensors are carried to comparator circuits, and the exit signals of these comparator circuits are offered via a logical circuit consisting of AND and OR ports as a warning signal to peripheral apparatus.

The logical signal is preferably circuited in such a manner that the exit signals of the comparator circuits for the absolute or relative deviation in the amplitude with which the signal from the sensors varies and for the phase difference of the signal from the sensors and a reference signal are offered to an OR port, the exit signal of which is offered to an AND port, to which AND port the exit signal from the comparator circuit for the absolute or relative deviation in the signal from the sensors is also offered.

The invention will now be explained further below with the aid of drawings.

Fig. 1 shows a device in schematic form for detecting air in the pleural cavity.

Fig. 2 shows a block diagram of the electronic circuit of a pneumothorax detector according to the invention.

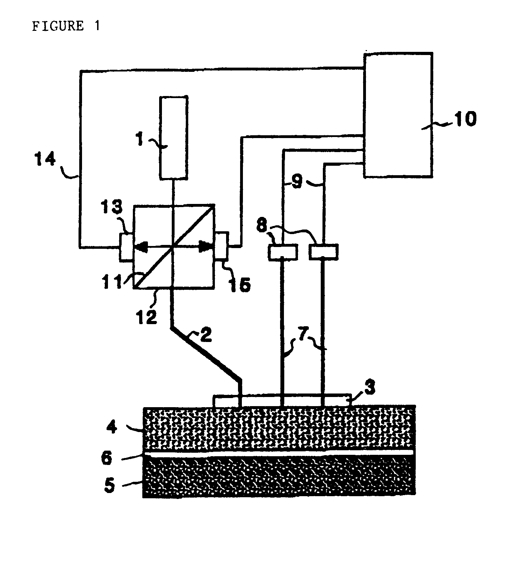

Fig. 1 provides a schematic display of a device for detecting air in the pleural cavity, with a light source (1) which emits light via a photo-conductive fibre (2) which is attached in a container (3) onto a place on the thorax wall (4). The light which is scattered from lung tissue (5) and pleural cavity (6) is led via one or more fibres (7), which are also attached in the holder (3) to one or more sensors (8). The signals (9) from the sensors (8) are offered to an electronic device (10) to be processed. The figure also shows a semi-silvered mirror (11) in a housing (12), in which a sensor (13) is situated, the signal (14) of which is offered to the electronic processing unit (10). The latter signal (14) can, in combination with a frequency modulating light source, be offered in a known manner to a lock-in amplifier, in order to exclude the influence of disturbing frequencies (for example artificial light from the surroundings).

Instead of, or as well as, the photo-conducting fibres (7) which lead to the sensors (8) use can also be made for the conduction of the light which is to be detected of the same fibre (2) along which the emitted light is conducted, in combination with a semi-silvered mirror (11) and a sensor (15).

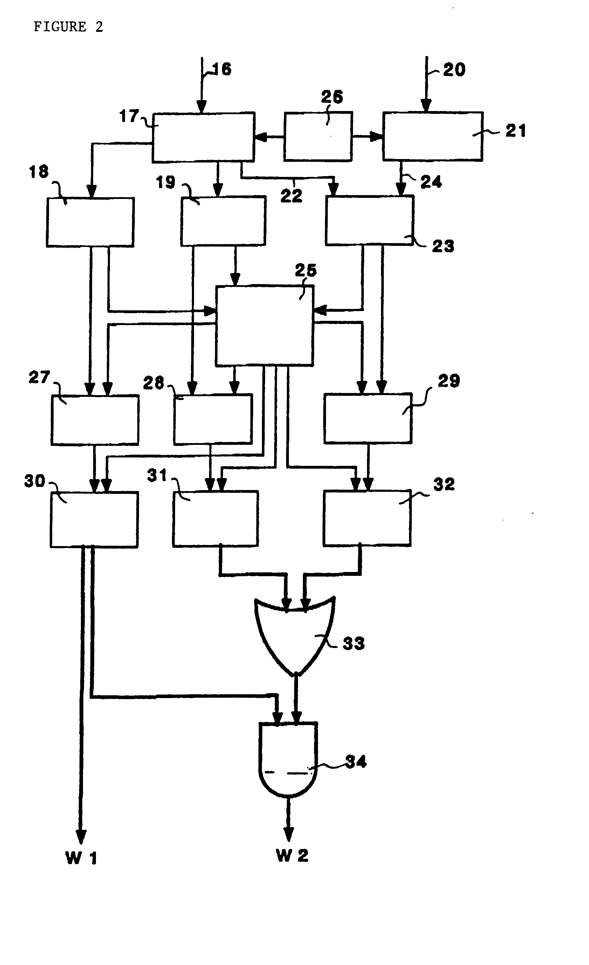

Fig. 2 shows a block diagram of the electronic circuit of a pneumothorax detector which contains a device for detecting air in the pleural cavity according to the invention. The signal (16) from the sensor is offered (after possible lock-in detection) via an analogue/digital (A/D) converter (not shown here) and via a circuit (17) in which it is marked by the time of a real-time clock (RTC) (26) to two function value generator circuits (18,19), in which numeric values S and A are allocated to, respectively, the average size of the signal from the sensor and the amplitude with which the signal from the sensor varies. A digitalized signal (20) which is deduced from the respiratory frequency is also marked with the time of the RTC (26) in a circuit (21), and offered together with the sensor signal (22) to a function value generator (23) in which a numeric value F is allocated to the phase difference of sensor and reference signal (22 and 24). The values S, A and F which are measured upon commencement, and which correspond with the physiological situation of the patient in which no pneumothorax has yet developed, are stored in a memory (25). The signals S, A and F are offered to a circuit in which the values of the deviation $\triangle S$ and $\triangle A$ in the average values of S and A and the change $\triangle F$ in the phase difference F are determined. In comparator circuits (30, 31, 32) the values $\triangle S$, $\triangle A$ and $\triangle F$ are compared with threshold values which are determined by empirically acquired standard values of $\triangle S$, $\triangle A$ and $\triangle F$, which are stored in the memory (25). The exit signal of the comparator circuits (31, 32) for $\triangle A$ and $\triangle F$ are offered via an OR port (33) to an AND port (34), as is the exit signal of the comparator circuit (20) for $\triangle S$.

In the event of a change in the size of the sensor signal a warning signal $W_1$ is generated. This signal change occurs in the case of a pneumothorax, but can also have other causes for example a too strong movement of the patient concerned). Warnings due to other causes can, under certain circumstances, also be desired and/or necessary. The development of a pneumothorax leads to a change in the signal size which is accompanied by a change of the amplitude with which the sensor signal varies or a change in the phase difference between the sensor signal and reference signal. In that case a second warning signal $W_2$ is generated.

## Claims

1.  Device for detecting air in the pleural cavity of a human being or an animal,
    characterized in that this contains at least one light source which emits light onto one or more places on the outside of the chest wall situated in front of the pleural cavity, directly or indirectly via a photo-conductive fibre or an imaging system, which light is scattered and reflected in and from the edge surfaces of the pleural cavity, as well as one or more photo-sensitive sensors to detect the scattered and reflected light at one or more places on the outside of the chest wall, as well as an electronic device for processing the signals emanating from the sensors.

2. Device according to claim 1,
characterized in that the wave length of the light emitted by the light source amounts to a minimum of 550 nanometres and a maximum of 1200 nanometres.

3. Device according to one of the claims 1 or 2,
characterized in that the light source is a semi-conductor light source, especially a semi-conductor laser.

4. Device according to one of the claims 1-3,
characterized in that a sensor and a light source, or the ends of the photo-conducting fibres or imaging systems connected to that sensor and that light source, are combined in a holder.

5. Device according to claim 4,
characterized in that the distance between the sensor and the light source, or, respectively, the tips of the fibres or the imaging systems, amounts to a maximum of 2 cm, or that the tips coincide.

6. Device according to one of the claims 1-5,
characterized in that the electronic device contains a circuit with which the absolute and/or relative deviation of the average value as time progresses of the amplitude with which the signal of the sensors varies is directly determined.

7. Device according to one of the claims 1-5,
characterized in that the electronic device contains a circuit with which the absolute and/or relative deviation of the average value as time progresses of the signal from the sensors is directly determined.

8. Device according to one of the claims 1-5,
characterized in that the electronic device contains a circuit with which the frequency of the signals from the sensors is determined.

9. Device according to one of the claims 1-5,
characterized in that the electronic device contains a circuit with which the phase difference between the signal from the sensors and a reference signal is determined.

10. Apparatus for detecting a pneumothorax, based on detection of air in the pleural cavity, characterized in that this contains a device according to one or more of the claims 6 up to and including 9.

11. Apparatus for detecting a pneumothorax, based on detection of air in the pleural cavity, characterized in that this contains a device according to the claims 2 up to and including 7 and 9.

12. Apparatus according to claim 11,
characterized in that the value of the phase difference of the signal from the sensors and a reference signal, the value of the absolute and/or relative deviation in the amplitude with which the signal from the sensors varies and the value of the absolute and/or relative deviation in the average signal from the sensors is carried to comparator circuits, and offered via a logical circuit consisting of AND and OR ports as a warning signal to peripheral apparatus.

13. Apparatus according to claim 12,
characterized in that the exit signals from the comparator circuits for the absolute or relative deviation in the amplitude with which the signal from the sensors varies and for the phase difference of the signal from the sensors and a reference signal are offered to an OR port, the exit signal of which is offered to an AND port, to which AND port the exit signal from the comparator circuit for the absolute or relative deviation in the average signal from the sensors is also offered.

FIGURE 1

FIGURE 2

European
Patent Office

EUROPEAN SEARCH
REPORT

Application Number

EP 91 20 1551

# DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | GB-A-2 057 886   (V. PUCHOLT) <br> * the whole document * <br> – – – | 1 | A 61 B 5/00 |
| Y,A | US-A-4 269 192   (K. MATSUO) <br> * column 2, lines 28 - 62 * * column 3, lines 21 - 55; figures * <br> – – – | 1,4-7 | |
| A | EP-A-0 359 433   (POLARTECHNICS, LTD.) <br> * column 3, line 18 - column 5, line 47 * <br> – – – | 2-7 | |
| A | US-A-4 449 535   (G. RENAULT) <br> * column 2, line 49 - column 3, line 61 * * column 8, lines 16 - 47; figures * <br> – – – – – | 2,4-7 | |

TECHNICAL FIELDS
SEARCHED (Int. Cl.5)

A 61 B

The present search report has been drawn up for all claims

| Place of search | Date of completion of search | Examiner |
|---|---|---|
| The Hague | 27 August 91 | RIEB K.D. |